# EUROPEAN PATENT SPECIFICATION

(11) **EP 2 465 568 B1**
(45) Date of publication and mention of the grant of the patent: **10.08.2016**
(21) Application number: 11194079.7
(22) Date of filing: 16.12.2011
(51) Int. Cl.: A61M 25/01

(54) **Steerable guide catheter having preformed curved shape**
Steuerbarer Führungskatheter mit vorgeformter Kurvenform
Cathéter guide orientable doté d'une forme incurvée préformée

(30) Priority: 19.12.2010 US 201061424658 P
(43) Date of publication of application: 20.06.2012
(73) Proprietor: Mitralign, Inc., Tewksbury, MA 01876 (US)
(72) Inventor: Morrill, Richard J., North Billerica, MA Massachusetts 01862 (US)
(74) Representative: Johnson, Richard Alan

(56) References cited:
- EP-A2- 0 521 595
- WO-A1-01/03766
- WO-A1-02/30310
- WO-A1-2009/137712
- US-A- 5 662 606
- US-A1- 2006 025 750
- US-A1- 2010 185 172

## Description

### TECHNICAL FIELD

The present invention relates to tissue fastening, and in particular, to a tissue fastening performed in a minimally invasive and percutaneous manner using hand operated instruments including a steerable guide catheter that is configured to deliver a guide wire to the annulus tissue of the mitral valve to allow placation thereof. The steerable guide catheter can also be used in additional applications unrelated to tissue fastening as discussed herein.

### BACKGROUND

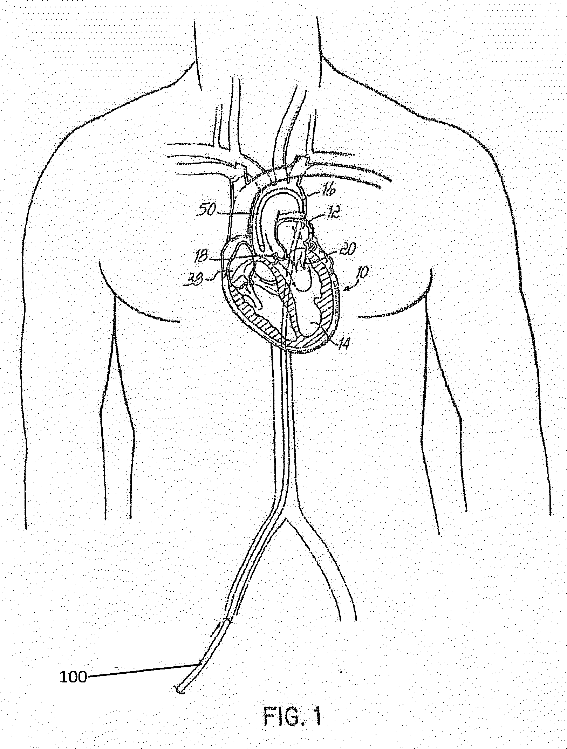

Referring initially to Figs. 1-4 solely for purposes of understanding the anatomy of a heart 10, and specifically the left side of the heart 10, the left atrium (LA) 12 and left ventricle (LV) 14 are shown. An aorta 16 receives oxygenated blood from left ventricle 14 through an aortic valve 18, which serves to prevent regurgitation of blood back into left ventricle 14. A mitral valve 20 is positioned between left atrium 12 and left ventricle 14, and allows one-way flow of the oxygenated blood from the left atrium 12 to the left ventricle 14.

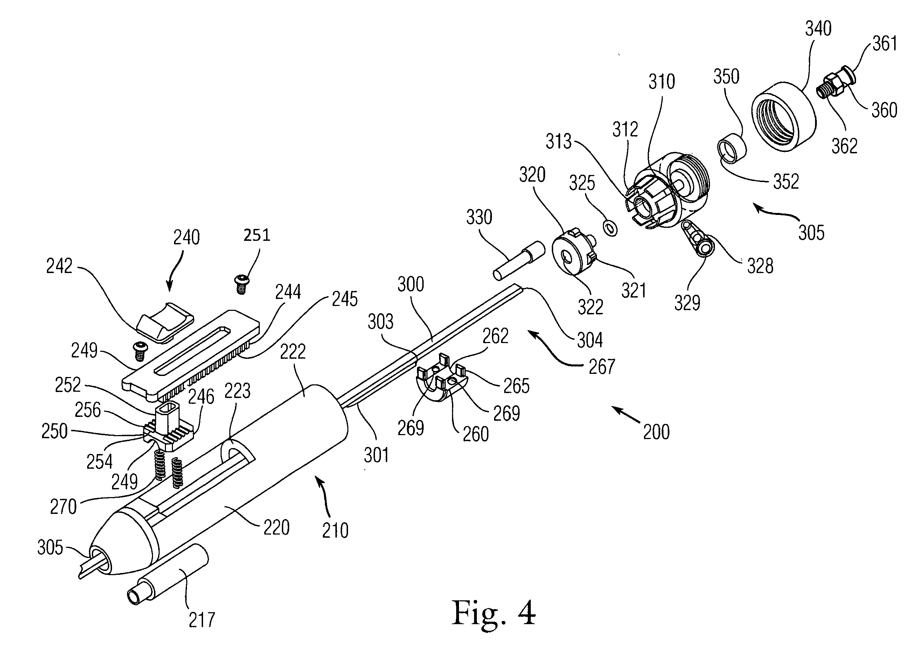

Mitral valve 20, which will be described below in more detail, includes an anterior leaflet 22 and a posterior leaflet 24 that are coupled to cordae tendonae 26, 28 (Fig. 4). Cordea tendonea 26, 28 serve as "tension members" that prevent the leaflets 22, 24 of mitral valve 20 from moving past their closing point and prolapsing back into the left atrium 12. When left ventricle 14 contracts during systole, cordae tendonae 26, 28 limit the upward motion (toward the left atrium) of the anterior and posterior leaflets 22, 24 past the point at which the anterior and posterior leaflets 22, 24 meet and seal to prevent backflow from the left ventricle 14 to the left atrium 12 ("mitral regurgitation" or "mitral insufficiency"). Cordae tendonae 26, 28 arise from a columnae carnae or, more specifically, a musculi papillares (papillary muscles) of the columnae carnae. In various figures herein, some anatomical features have been deleted solely for clarity.

Anterior leaflet 22 and posterior leaflet 24 of the mitral valve 20 are generally thin, flexible membranes. When mitral valve 20 is closed, anterior leaflet 22 and posterior leaflet 24 are generally aligned and contact one another along a "line of coaptation" several millimeters back from their free edges, to create a seal that prevents mitral regurgitation. Alternatively, when mitral valve 20 is opened, blood flows downwardly through an opening created between anterior leaflet 22 and posterior leaflet 24 into left ventricle 14.

Many problems relating to the mitral valve may occur and may cause many types of ailments. Such problems include, but are not limited to, mitral regurgitation. Mitral regurgitation, or leakage, is the backflow of blood from left ventricle 14 into the left atrium 12 due to an imperfect closure of mitral valve 20. That is, leakage often occurs when the anterior and posterior leaflets 22, 24 do not seal against each other, resulting in a gap between anterior leaflet 22 and posterior leaflet 24 when the leaflets are supposed to be fully coapted during systole.

In general, a relatively significant systolic gap may exist between anterior leaflet 22 and posterior leaflet 24 for a variety of different reasons. For example, a gap may exist due to congenital malformations, because of ischemic disease, or because the heart 10 has been damaged by a previous heart attack. Such a gap may also be created when congestive heart failure, e.g., cardiomyopathy, or some other type of distress which causes a heart 10 to be enlarged. Enlargement of the heart 10 can result in dilation (stretching) of the mitral annulus. This enlargement is usually limited to the posterior valve annulus and is associated with the posterior leaflet 24, because the anterior annulus is a relatively rigid fibrous structure. When the posterior annulus enlarges, it causes the posterior leaflet 24 to move away from the anterior leaflet 22, causing a gap during systole because the two leaflets no longer form proper coaptation. This results in leakage of blood through the valve 20, or regurgitation.

Blood leakage through mitral valve 20 generally causes a heart 10 to operate less efficiently, as the heart 10 pumps blood both out to the body via the aorta 16, and also back (in the form of mitral regurgitation) into the left atrium 12. Leakage through mitral valve 20, or general mitral insufficiency, is thus often considered to be a precursor to congestive heart failure (CHF) or a cause of progressive worsening of heart failure. There are generally different levels of symptoms associated with heart failure. These levels are classified by the New York Heart Association (NYHA) functional classification system. The levels range from a Class 1 level which is associated with an asymptomatic patient who has substantially no physical limitations to a Class 4 level which is associated with a patient who is unable to carry out any physical activity without discomfort and has symptoms of cardiac insufficiency even at rest. In general, correcting or reducing the degree of mitral valve leakage may be successful in allowing the NYHA classification grade of a patient to be reduced. For instance, a patient with a Class 4 classification may have his classification reduced to Class 3 or Class 2 and, hence, be relatively comfortable at rest or even during mild physical exertion. By eliminating the flow of blood backwards into the left atrium 12, therapies that reduce mitral insufficiency reduce the workload of the heart 10 and may prevent or slow the degradation of heart function and congestive heart failure symptoms that is common when a significant degree of mitral insufficiency remains uncorrected.

Treatments used to correct for mitral valve leakage or, more generally, CHF, are typically highly invasive, open-heart surgical procedures. In extreme cases, this may include implantation of a ventricular assist device such as an artificial heart in a patient with a failing heart. The implantation of a ventricular assist device is often expensive, and a patient with a ventricular assist device must be placed on extended anti-coagulant therapy. Anti-coagulant therapy reduces the risk of blood clot formation for example, within the ventricular assist device. Reducing the risks of blood clots associated with the ventricular assist device is desirable, but anti-coagulant therapies may increase the risk of uncontrollable bleeding in a patient, e.g., as a result of a fall.

Rather than implanting a ventricular assist device, bi-ventricular pacing devices similar to pacemakers may be implanted in some cases, e.g., cases in which a heart beats inefficiently in a particular asynchronous manner. While the implantation of a bi-ventricular pacing device may be effective, not all heart patients are suitable for receiving a bi-ventricular pacing device. Further, the implantation of a bi-ventricular pacing device is expensive, and is generally not effective in significantly reducing or eliminating the degree of mitral regurgitation.

Open-heart surgical procedures that are intended to correct for mitral valve leakage, specifically, can involve the implantation of a replacement valve. Valves from animals, e.g., pigs, may be used to replace a mitral valve 20 in a human. While a pig valve may relatively successfully replace a mitral valve, such replacement valves generally wear out, thereby requiring additional open surgery at a later date. Mechanical valves, which are less likely to wear out, may also be used to replace a leaking mitral valve. However, when a mechanical valve is implanted, there is an increased risk of thromboembolism, and a patient is generally required to undergo extended anti-coagulant therapies.

A less invasive surgical procedure involves heart bypass surgery associated with a port access procedure. For a port access procedure, the heart may be accessed by cutting between ribs or sometimes removing parts of one or more ribs, as opposed to dividing the sternum to open the entire chest of a patient.

One open-heart surgical procedure that is particularly successful in correcting for mitral valve leakage and, in addition, mitral regurgitation, is an annuloplasty procedure. During an annuloplasty procedure, a medical device such as an annuloplasty ring may be implanted surgically on the left atrial side of mitral annulus (i.e., generally the attachment location of the base of the mitral valve to the heart). The device reduces a dilated mitral valve annulus to a relatively normal size and, specifically, moves the posterior leaflet closer to the anterior leaflet to aid anteriorposterior leaflet coaptation and thus improve the quality of mitral valve closure during systole. Annuloplasty rings are often shaped substantially like the letter "D" to correspond to the natural shape of the mitral annulus as viewed from above. Typically, the rings are formed from a rod or tube of biocompatible material, e.g., plastic, that has a DACRON mesh covering.

In order for an annuloplasty ring to be implanted, a surgeon surgically attaches the annuloplasty ring to the mitral valve on the atrial side of the mitral valve. Conventional methods for installing a ring require open-heart surgery which involves opening a patient's sternum and placing the patient on a heart bypass machine. The annuloplasty ring is sewn on a top portion of the mitral valve. In sewing the annuloplasty ring onto the mitral valve, a surgeon generally sews the straight side of the "D" to the fibrous tissue located at the junction between the posterior wall of the aorta and the base of the anterior mitral valve leaflet. As the curved part of the ring is sewn to the posterior aspect of the annulus, the surgeon alternately acquires a relatively larger amount of tissue from the mitral annulus, e.g., a one-eighth inch (0.32cm) bite of tissue, using a needle and thread, compared to a relatively smaller bite taken of the fabric covering of the annuloplasty ring. Once the thread has loosely coupled the annuloplasty ring to the mitral valve annulus tissue, the annuloplasty ring is slid into contact with the mitral annulus. The tissue of the posterior mitral annulus that was previously stretched out, e.g., due to an enlarged heart, is effectively reduced in circumference and pulled forwards towards the anterior mitral leaflet by the tension applied by annuloplasty ring with the suture or thread. As a result, a gap between anterior leaflet 22 and posterior leaflet 24 during ventricular contraction or systole may be reduced and even substantially closed off in many cases thereby significantly reducing or even eliminating mitral insufficiency. After the mitral valve 20 is shaped by the ring, the anterior and posterior leaflets 22, 24 will reform typically by pulling the posterior leaflet 24 forward to properly meet the anterior leaflet 22 and create a new contact line that will enable mitral valve 20 to appear and to function properly.

Although a patient that receives an annuloplasty ring may be subjected to anti-coagulant therapies, the therapies are not extensive, as a patient is only subjected to the therapies for a matter of weeks, e.g., until tissue grows over the annuloplasty ring.

Another type of procedure that is generally effective in reducing mitral valve leakage associated with prolapse of the valve leaflets involves placing a single edge-to-edge suture in the mitral valve 20 that apposes the mid-portions of anterior and posterior leaflets 22, 24. For example, in an Alfieri stitch or a bow-tie repair procedure, an edge-to-edge stitch is made at approximately the center of the gap between an anterior leaflet 22 and a posterior leaflet 24 of a mitral valve 20. Once the stitch is in place between the anterior and posterior leaflets 22, 24, it is pulled in to form a suture which holds anterior leaflet 22 against posterior leaflet 24.

Another surgical procedure that reduces mitral valve leakage involves placing sutures along a mitral valve annulus around the posterior leaflet 24. These sutures may be formed as a double track, e.g., in two "rows" from a single strand of suture material. The sutures are tied off at approximately a central point (P2) of posterior leaflet 24. Pledgets are often positioned under selected sutures to prevent the sutures from tearing through annulus 40. When the sutures are tightened and tied off, the circumference of the annulus 40 may effectively be reduced to a desired size such that the size of a systolic gap between posterior leaflet 24 and an anterior leaflet 22 may be reduced.

While invasive surgical procedures have proven to be effective in the treatment of mitral valve leakage, invasive surgical procedures often have significant drawbacks. Any time a patient undergoes open-heart surgery, there is a risk of infection. Opening the sternum and using a cardiopulmonary bypass machine has also been shown to result in a significant incidence of both short and long term neurological deficits. Further, given the complexity of open-heart surgery, and the significant associated recovery time, people that are not greatly inconvenienced by CHF symptoms, e.g., people at a Class 1 classification, may choose not to have corrective surgery. In addition, people that need open heart surgery the most, e.g., people at a Class 4 classification, may either be too frail or too weak to undergo the surgery. Hence, many people that may benefit from a surgically repaired mitral valve may not undergo surgery.

In another method, a cinching device is placed within the coronary sinus (CS) using a catheter system, with distal, mid, and proximal anchors within the lumen of the CS to allow plication of the annulus 40 via the CS. In practice, these anchors are cinched together and the distance between them is shortened by pulling a flexible tensile member such as a cable or suture with the intent being to shorten the valve annulus 40 and pull the posterior leaflet 24 closer to the anterior leaflet 22 in a manner similar to an annuloplasty procedure. Unfortunately, since the tissue that forms the CS is relatively delicate, the anchors are prone to tear the tissue during the cinching procedure. In addition, the effect on the mitral annulus may be reduced when the CS of a particular patient is not directly aligned with the mitral annulus. Other minimally invasive techniques have been proposed but have various drawbacks related to such factors as effectiveness and/or accuracy of catheter-based implementation.

US 2010/185172 discloses a diagnostic catheter having multiple curve regions, with at least two of the curve regions defining different planes.

WO 2002/030310 discloses a steerable electrophysiology catheter having manipulators to enable deflection of a distal segment of a distal tip section of the catheter body.

### SUMMARY

According to the invention, there is provided a steerable guide catheter as set out in claim 1.

In one embodiment, the steerable guide catheter includes a handle and an elongated shaft extending outwardly away from and being coupled to the handle. The shaft has a distal end opposite the handle. The shaft further has a first preformed curved section at the distal end and a second preformed curved section that is located proximal to the first preformed curved section. The two preformed curved sections can be fabricated as part of a molding process whereby the two preformed curved sections are effectively "baked in" the catheter shaft.

The guide catheter also has a steering mechanism for controllably steering at least the first preformed curved section at the distal end. The steering mechanism is coupled between the handle and elongated shaft and being configured such that actuation of the steering mechanism causes the first preformed curved section to be bent in at least two planes. The two planes are offset from one another resulting in the first preformed curved section moving in a corkscrew shape manner upon actuation of the steering mechanism.

As discussed herein, the steerable guide catheter can be used in other applications in which a material and/or instrument is delivered to target tissue for providing localized therapy using surrounding tissue.

These and other aspects, features and advantages shall be apparent from the accompanying drawings and description of certain embodiments of the invention.

### BRIEF DESCRIPTION OF THE DRAWINGS

Fig. 1 is a schematic illustration of a patient with the anatomy of the heart in cross section and a steerable guide obturator introduced through the vascular system into the aorta and heart of the patient;
Fig. 2 is a cross sectional view of the heart from above showing the introduction of a steerable guide catheter, in accordance with one embodiment of the present invention, through the obturator of Fig. 1;
Fig. 3 is a side elevation view of an exemplary obturator;
Fig. 4 is an exploded perspective view of a steerable guide catheter according to one exemplary embodiment;
Fig. 5 is a cross-sectional view of an actuator that is part of the guide catheter of Fig. 4;
Fig. 6 is an end view of the steerable guide catheter;
Fig. 7 is a cross-sectional view taken along the line 7-7 of Fig. 6;
Fig. 8 is a side view showing the inner components of the handle body of the steerable guide catheter;
Fig. 9 is a top plan view of the handle body;
Fig. 10 is a side elevation view of an exemplary catheter shaft;
Fig. 11 is an enlarged, close-up side view of a distal tip of the catheter shaft;
Fig. 12 is a cross-sectional view showing the lumens of the catheter shaft;
Fig. 13 is a side elevation view of a distal end section of the catheter shaft in a normal, rest position prior to actuation of the steering mechanism;
Fig. 14 is side elevation view of a mandrel for forming a first preformed curved section (distal tip section) of the catheter;

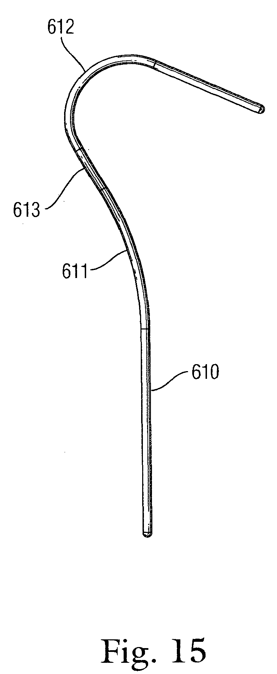

Fig. 15 is a side elevation view of a mandrel for forming a second preformed curved section of the catheter;
Fig. 16 illustrates an articulated arch curve template for the catheter shaft;
Fig. 17 illustrates an apex curve template as baked (rest position) and as articulated; and
Fig. 18 is a side elevation view of the catheter shaft in an elongated orientation showing the steering wire exiting one lumen (steering wire lumen) and having a corkscrew orientation and being attached to the main lumen.

### DETAILED DESCRIPTION OF CERTAIN EMBODIMENTS OF THE INVENTION

Reference will be made to the various figures in describing the methods, devices and systems in various forms useful to the purpose of plicating tissue, for example, and particularly useful for plicating annulus tissue associated with the mitral valve of a patient. It will be appreciated that although specific details of the methods, devices and systems will be given herein, many different changes, substitutions and additions may be made to such details by those of ordinary skill while still falling within the inventive aspects more generally set forth herein and understood by those of ordinary skill upon review of the present disclosure in its entirety. It should be noted that the terms "proximal" and "distal" are used, as conventional in the art, to denote spatial relationship relative to the person using the particular device or component. That is, "proximal" refers to a position closer to the user and "distal" refers to a position farther from the user.

While the devices are discussed at length in terms of being used to plicate tissue, it will be understood and appreciated that the devices can be used in other applications not related to plicating tissue. For example and as discussed below, the instruments can be used to correct other abnormalities and also can be used to deliver material, such as stem cells, nuclear material, molecules, etc., to a target site for providing remedial treatment.

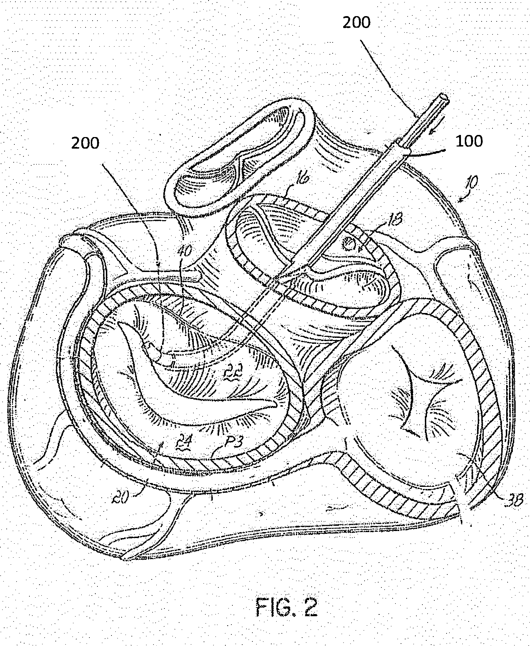

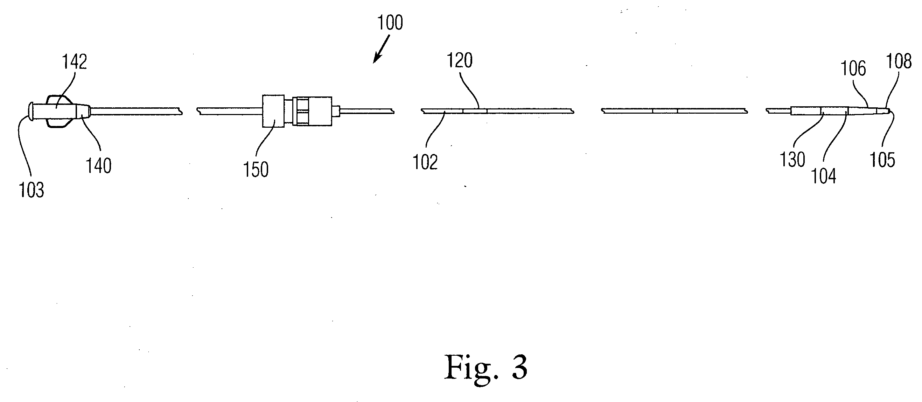

Figs. 2 and 3 illustrate a steerable guide obturator 100 according to one embodiment. The obturator 100 has an elongated obturator body 102 extending along a longitudinal, central axis and including a proximal end 103 and a distal end 105. The obturator body 102 can include a flexible, omnidirectionally deflectable distal end section 104 terminating at a distal tip 106 that can have a curved or rounded outer tip surface 108. It will be appreciated that the distal end section 104 of the obturator body 102 can be a short section that is more flexible than the remainder of the obturator body 102.

The obturator 100 includes a main shaft 120 that extends the length of the obturator and the distal tip 106 can be attached to the main shaft 120 using conventional techniques including the use of an adhesive (medical grade adhesive) and also the use of an anchor ban 130 (platinum iridium anchor band). The main shaft 120 can be in the form of a braided shaft. The main shaft 120 is a hollow structure with a central lumen extending therethrough.

The obturator 100 includes a hub 140 that is disposed at the proximal end 103 of the obturator body 102 and can be attached to one end of the main shaft 120 using conventional techniques, including using an adhesive, such as an epoxy material. The hub 140 is an at least partially hollow member and includes a central bore that is axially aligned with the central lumen of the main shaft 120. The hub 140 can include wing-like structures 142 (e.g., two opposing wings) that permit the hub 140 to be easily grasped.

The obturator 100 also includes a touhy adaptor 150 that is disposed along the length of the obturator body 102. The touhy adaptor 150 has a body (such as a PVC body) and an internal gasket. The internal gasket is chemically resistant, prevents backflow of fluids and secures the position of a guidewire or instrument when closed. As shown, the touhy adaptor 150 is disposed between two different sections of the main conduit 120 and thus, one section of the main conduit 120 extends between the hub 140 and one end of the touhy adaptor 150, while another section of the main conduit 120 extends between the other end of the touhy adaptor 150 and the tip 106.

The central lumen of the obturator 100 provides a passageway for a catheter guide wire, or a contrast dye, or an imaging probe such as a fiber optic bundle or an ultrasonic probe, etc. As described below, the obturator 100 is described in combination with a guide wire and is used as part of a surgical procedure that is described in detail below.

The obturator 100 can be of a steerable type in that within the obturator body, a conventional steering mechanism (not shown) can be disposed and operatively connected to an actuator to permit steering of the obturator 100. For example, the steering mechanism can include one more steering wires that permit the position of the tip 106 and the shape of the shaft 120 to be changed in order to allow the obturator tip 106 to be placed at the target location.

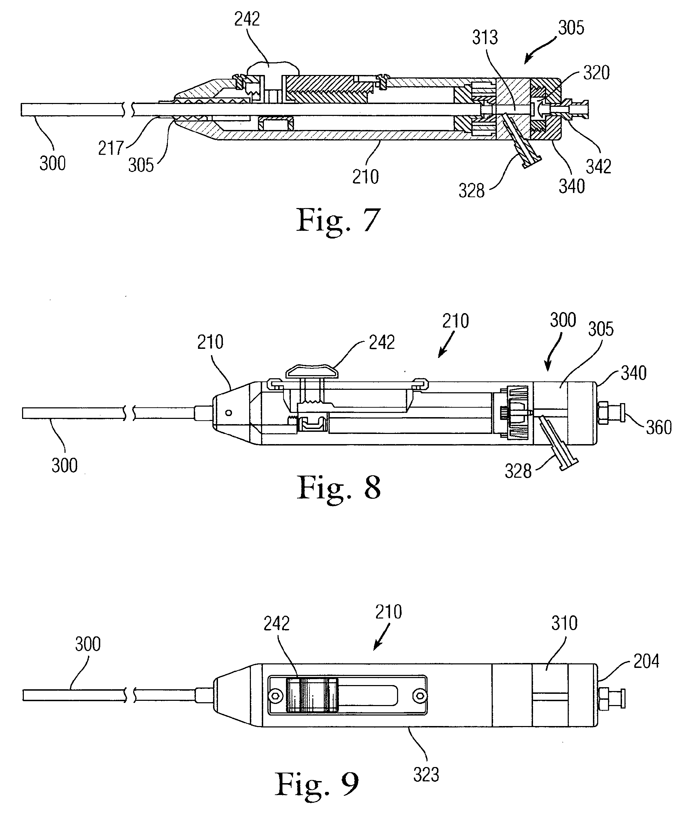

In accordance with the present invention, a steerable guide catheter 200 is provided and shown in Figs. 4-12. The guide catheter 200 is an elongated instrument having a first (distal) end 202 and an opposing second (proximal) end 204. A handle 210 is disposed at the proximal end 204 and is configured to allow for steering of the guide catheter 200 as described herein. The guide catheter 200 also includes an elongated catheter shaft 300 that has a distal end 302 and an opposing proximal end 304 that mates with the handle 210. In particular, the proximal end 304 is received through a forward opening 305 that is formed in the handle 210. A bushing 217 can be provided in the handle body at the forward opening 305 and is configured to permit the guide catheter 200 to be routed through the bushing 217. The proximal end 304 of the catheter shaft 300 is thus disposed within the handle 210 and is coupled to other working components of the handle 210 as described below. A set screw can be used to set the bushing 217 in place.

As mentioned above, the catheter shaft 300 of the guide catheter 200 includes a steering mechanism which can be in the form of a steering guide wire 301 that is disposed along a length of the catheter shaft 300 and is manipulated to cause the shaft 300 to move (e.g., to bend in a curling motion). The details of the routing of the steering guide wire 301 along the catheter shaft 300 are described in greater detail below. While the guide wire 301 is for the most part fully contained within the catheter shaft 300, one end 303 of the guide wire 301 extends through an opening formed in the catheter shaft 300 and runs along externally along a length of the catheter shaft 300. This end 303 of the guide wire 301 is accessible and therefore, the end 303 can be pulled or pushed which results in controlled movement of the catheter shaft 300.

Fig. 4 illustrates the various components of the handle 210. More specifically, the handle 210 includes a handle body 220. The illustrated handle body 220 has a cylindrical shape and is hollow to permit reception of other working components of the handle 210. Along a top 222 of the body 220, a slot 223 is formed to allow an actuator 240 to extend therethrough and be accessible to an operator/user. The illustrated actuator 240 is a slider that is moved linearly (forward and backward) to cause movement and steering of the catheter shaft 300.

The actuator 240 includes a button 242, a handle rack 244 and a button rack 246. The button 242 has a concave top surface that is contoured to receive a thumb of the user to allow the user to grasp the handle body 220 and slidingly move the actuator 240. The button 242 has a post or protrusion 243 that extends downwardly from a bottom surface of the button. The handle rack 244 is in the form of a block, such as a rectangular block, that has teeth 245 formed along a bottom surface 247 of the handle rack 244. The handle rack 244 is received within the slot 223 formed in the body 220. The handle rack 244 includes a slot 249 that is formed centrally within the rack 244. The illustrated slot 249 has a rectangular shape. The handle rack 244 can be coupled to the handle body 220 using conventional means including fasteners 251, such as screws, at each end of the rack 244. The handle rack 244 is thus fixed and stationary relative to the handle body 220.

The button rack 246 includes a base portion 250 and a post portion 252 that extends upwardly from the base portion 250. The post portion 252 is centrally located relative to the base portion 250. The post portion 252 thus partitions the base portion 250 such that two rack sections 254 are formed on either side of the post portion 252. Each rack section 254 includes teeth 256 formed along a top surface of the base portion 250. The teeth 256 are complementary to the teeth 245 and in particular, the teeth 256, 245 are designed to intermeshingly mate with one another, thereby coupling the movable button rack 246 to the fixed handle rack 244. The mating between the teeth 256, 245 permit the button rack 246 to be fixed (locked) in place along the handle rack 244 at a particular location, while still permitting the button rack 246 to be moved along the handle rack 244 by disengaging the button rack 246 from the handle rack 244 as discussed below.

The post portion 252 of the button rack 246 is a hollow member that receives the post 243 of the button 242 to thereby couple the button 242 to the button rack 246 so that movement of the button 242 is translated into movement of the button rack 246. The button 242 is located on the exterior of the handle rack 244 and the button rack 246 is located internally within the handle on the other side of the handle rack 244. The bottom surface of the button rack 246 has a channel 249 formed therein along the length thereof. The channel 249 receives the catheter shaft 300 and permits the button rack 246 to slidingly travel over the catheter shaft 300.

The actuator 240 also includes slider rack (a shuttle) 260 that is coupled to the button rack 246. The slider rack 260 includes a top surface that includes a channel 262 that is identical or similar to the channel 249 and receives the catheter shaft 300 to permit the slider rack 260 to slidingly travel over the catheter shaft 300. Thus, the button rack 246 is disposed on one side of the catheter shaft 300, while the slider rack 260 is disposed on the other opposite side of the catheter shaft 300. The slider rack 260 can be coupled to the button rack 246 using conventional techniques and in particular, a mechanical attachment can be formed between the two members. For example, the slider rack 260 can include locking tabs 265 that are received within complementary openings formed in the button rack 246 or fasteners can be used to couple the two together.

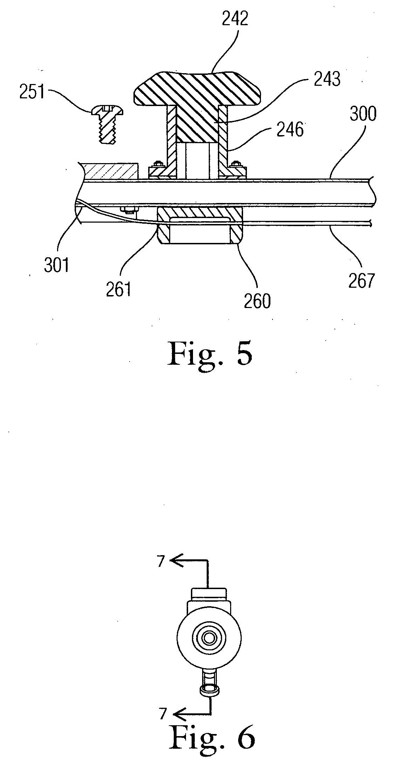

The steering wire 301 is fixedly attached to the sliding components of the actuator 240 such that movement of the actuator 240 within the handle is translated into movement (a pulling or pushing motion) of the steering wire 301. For example, the steering wire 301 can be fixedly attached to the slider rack 260 and as illustrated, the steering wire 301 passes through an opening 261 formed in the slider rack 260. The opening 261 extends completely through the slider rack 260 and as shown in Fig. 5, the steering wire 301 passes through slider rack 260 and exits the opening 261 at one end of the slider rack 260. A coupling member, such as a fitting, 267 can be used to effectively fix the steering wire 301 to the slider rack 260. The fitting 267 can be a hollow member that receives the end of the steering wire 301 and is then attached to the wire 301, using conventional means (e.g., crimping). Since the fitting 267 has dimensions greater than the opening 261, the fitting 267 cannot be received within the opening 261, thereby fixing the wire 301 to the slider rack 260.

The top surface of the slider rack 260 includes a pair of holes or recesses 269 that are formed on either side of the channel 262. The illustrated slider rack 260 generally has a semi-circular shape that rests against the complementarily shaped hollow interior of handle 210 to allow sliding movement of the slider rack 260. Similarly, the bottom surface of the button rack 246 includes a pair of holes or recesses (not shown) that are axially aligned with the holes 269 when the button rack 246 and slider rack 260 mate together. A biasing element 270 is provided between the button rack 246 and the slider rack 260 and for applying a force to the button rack 246 so as to place the teeth 256 into engagement with the teeth 245. The biasing element 270 thus causes the button rack 246 to be interlockingly mated with the handle rack 244. Thus, in a normal, rest position, the button rack 246 is biased upward into engagement with the handle rack 244.

The biasing element 270 can be in the form of a pair of springs that are securely received within the holes formed in the underside of the handle rack 244 and the holes in the top surface of the button rack 246. The springs 270 thus bias the button rack 246 into contact with the handle rack 244. To disengage the movable button rack 246 from the fixed handle rack 244, the button 242 is pressed down causing the button rack 246 to move downward causing the springs 270 to compress and store energy. When the button rack 246 moves down and the springs 270 store energy, the teeth 245, 256 disengage from one another, thereby permitting the actuator 240 to freely move along the handle. This movement (linear) of the actuator 240 causes the steering wire 301 to be moved accordingly, thereby resulting in the catheter shaft 120 assuming a more curved (bent) configuration or a more straightened configuration.

The actuator 240 travels along the catheter shaft 200 and the degree of travel of the actuator 240 is limited by the length of the slot 223 formed in the handle body 220.

The handle 210 also includes a rotator assembly 305 that is disposed at the end proximal end of the handle 210 and is rotatably coupled thereto. The proximal end 304 of the catheter shaft 300 is coupled to the rotator assembly 300 in such away that the rotator assembly 305 can rotate about the catheter shaft 200. The rotator assembly 305 includes a rotator body 310 that has a central bore 313 formed therein and is open at both ends of the rotator body 310. The illustrated rotator body 310 has a cylindrical shape that complements the cylindrical shape of the handle body. An end 312 of the rotator body 310 can have a structure to permit the rotator body 310 to mate with the open end of the handle body and in particular, the end 312 can have a flange-like structure that is received within the opening of the handle body. The flange-like structure at the end 312 of the rotator body 310 can mate with the end of the handle body in such a way that the rotator body 310 is securely attached to the handle body.

The illustrated rotator body 310 further includes a side port 328 that extends outwardly from the rotator body 310 at a prescribed angle. The side port 328 is a hollow structure and includes a bore 329 that is communication with the center bore 313. The side port 328 can thus be used to introduce or remove an element into the center bore 313. The side port 328 can be in the form of a flush port and can be a female luer type connector that mates with a complementary male connector.

In one embodiment, the rotator body 310 is formed of a transparent material to permit the user to see within the central bore 313 to ensure that the guide catheter 100 is operating properly. Since the bore 313 is in communication with the central lumen formed in the catheter shaft 300, the user can see the object that is fed into and through the guide catheter 200. The transparent rotator body 310 allows the user to detect air bubbles, fluid flow, etc., that may be harmful.

The rotator assembly 305 includes a means for connecting the proximal end 304 of the catheter shaft 300 such that the catheter shaft 120 is fixedly attached to the handle body, while the rotator body 310 and handle body are free to rotate relative to one another. The rotator assembly 305 can include a proximal insert 320 that includes a center bore or opening 322 formed therein. The bore 322 is sized so that the catheter shaft 300 can be received through the bore 322. The catheter shaft 300 can be fitted to a length of tubing 330 that terminates the proximal end of the catheter shaft 300 and is configured so that it can be received within the bore 320. The hollow tubing 330 thus provides a lumen that is axially aligned with the lumen of the catheter shaft such that an object being inserted through the tubing 330 is fed into the center lumen of the catheter shaft 300. The insert 320 also mates with one end of the rotator body 310 and the bore 313 of the rotator body 310 is axially aligned with the bore (lumens) of both the insert 320 and the catheter shaft 120. The insert 320 is coupled to the rotator body 310 such that the two rotate together.

As shown, the insert 320 includes a protrusion at one end that mates with a complementary feature of the rotator body 310, such as a slot at the end of the rotator body 310. A seal, such as an O-ring, 325 can be disposed between the protrusion 321 and the protrusion of the rotator body 310.

The rotator assembly 300 also includes a proximal handle cap 340 that is fixedly coupled to one end of the rotator body 310. The cap 340 is a hollow structure that includes an opening 342 formed therein. The opening 342 can be a circular shaped opening. The cap 340 can have inner threads 344 that permit the cap 340 to be securely attached to the rotator body 310 such that the cap 340 and rotator body 310 rotate together.

A wiper seal 350 can be disposed between the rotator body 310 and the cap 340. One end of the rotator body 310 can include a recess that receives and contains the wiper seal 350. The wiper seal 350 can be in the form of a cylindrical member that has a central bore or openings 352 that is axially aligned with the other center bores when the cap 340 and rotator body 310 mate together with the wiper seal 350 being disposed therebetween.

The opening 342 of the cap 340 can be a threaded opening and can receive a proximal luer 360 that represents the most proximal component and the proximal end of the guide catheter 100. The proximal luer 360 includes threads 362 that serve to securely attach the luer 360 to the cap 340 such that a portion of the luer 360 extends outwardly from the end of the cap 340. The luer 360 is a hollow structure with a bore or through hole 361 formed therethrough that permits an object, such as a wire or catheter, to be inserted into the hole 361 and fed through the lumen structure of the rotator assembly 300 and into the center lumen of the catheter shaft 300. The transparent nature of the rotator body 310 permits the object inserted into the luer 360 to be visible.

As described in more detail below, the rotator assembly 305 represents a portion of the guide catheter 100 that is held (gripped) when the user wishes to rotate the catheter shaft 300. To rotate the guide catheter 100, the handle body 210 is rotated in a prescribed direction while the rotator assembly 305 is held stationary. Since the catheter shaft 300 is fixedly attached to the handle body 210, the rotation of the handle body 210 is translated into rotation of the catheter shaft 300. This permits the tip of the catheter shaft 300 to be positioned at a desired position during the surgical procedure. This structural arrangement is particularly advantageous because it allows rotation of the catheter shaft 300 while assembly 305 and, in particular, any objects that are disposed within side port 320, are isolated from the rotational movement.

The handle 210 and its related components, such as the rotator assembly 305, can be formed of any number of different materials, including suitable plastics.

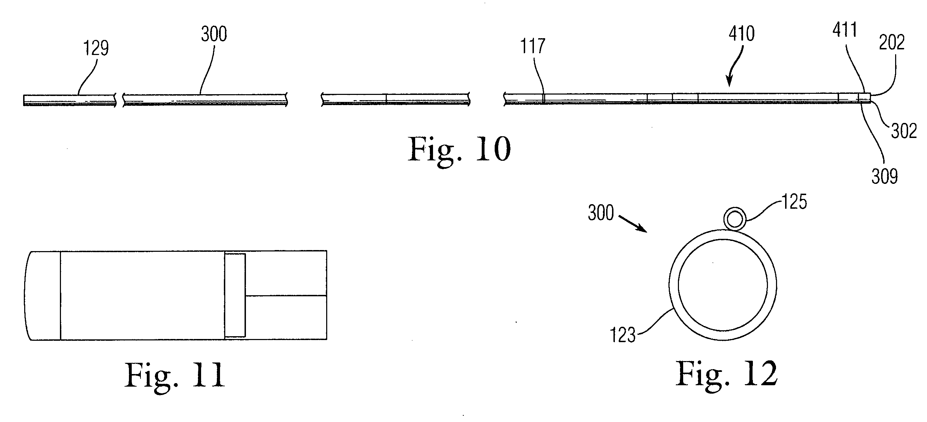

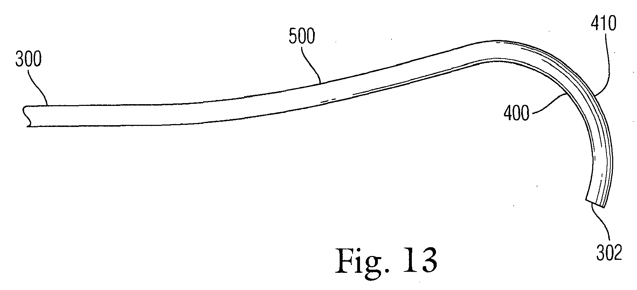

In accordance with the present invention, the catheter shaft 300 of the steerable guide catheter 100 is preformed to have a desired curved shape and more particularly, the catheter shaft 300 has a first preformed curved section 400 and a second preformed curved section 500 that is spaced from the first preformed curved section 400. Fig. 10 shows the catheter shaft 300 prior to the formation of the two preformed curved sections and Fig. 13 shows the two curved sections 400, 500 during a normal rest position prior to manipulating the steering mechanism. The first preformed curved section 400 is located at the distal end 302 of the catheter shaft 300, while the second preformed curved section 500 is proximally spaced from the first preformed curved section 400. The second preformed curved section 500 can be thought of as an aortic curve, while the first preformed curved section 400 can be thought of as a curved distal tip section 410 that is intended for placement between the papillary muscles and against the left ventricle wall under the mitral valve. The radius of curvature of the first preformed curved section 400 is greater than the radius of curvature of the second preformed curved section 500.

As described in more detail herein, the presence of two curves in the catheter shaft of the present invention provides a number of advantages and in particular, the two curves (curved sections 400, 500) advantageously allows the catheter 100 to better conform to the anatomy of the heart that is encountered during a typical surgical procedure performed to the annulus tissue of the mitral valve. More specifically, one exemplary surgical procedure that is described herein involves a retrograde passage of the catheter through the aortic valve, through the papillary muscles, to position the distal end of the catheter below the posterior side of the mitral valve.

The second preformed curved section 500 (aortic curved section) in the catheter 100 allows for less trauma to the tissue of the aortic arch. This secondary curve (second preformed curved section 500) increases manueverability and allows greater torque transmission in the catheter. This is because the curve reduces the amount of contact the catheter 100 has with the aorta. The curve allows the catheter to "bank off' of the aortic arch and allow optimal positioning and placement of the catheter. Instead of the entire length of the catheter resting against and following the curve of the aorta, forming the catheter 100 of the present invention with the secondary curve (curved section 500) reduces the length of the catheter that is contact with tissue in that region. Thus, it is easier to torque the catheter 100 because of less constriction.

The distal end curve (first preformed curved section 400) is designed to allow the catheter 100 to seat against the left ventricle wall while other medical devices are advanced through the central lumen of the catheter shaft to operate upon and/or penetrate the mitral annulus. The distal curve (first preformed curved section 400) allows the catheter 100 to seat against the wall as a support for the catheter (e.g., the catheter is banked off the wall and rests against it). While the catheter 100 is resting against the wall, the anchors/guide wires can be penetrated through the annulus.

In accordance with one embodiment and as described in more detail below, the first preformed curved section 400 is a steerable section in that the curvature thereof can be changed based on the user manipulating the actuator 240 that is part of the handle body 210. In contrast, the curvature of the second preformed curved section 500 is not substantially changed during use of the guide catheter 100 and therefore, the second preformed curved section 500 can be thought of as a fixed curvature section. The curved sections 400, 500 are described in greater detail below.

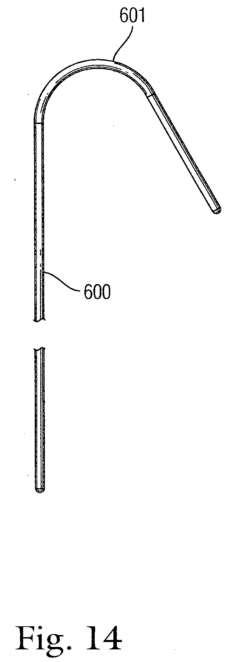

The first and second curved sections 400, 500 are preformed using conventional techniques, including but not limited to a molding operation. Fig. 14 shows a mandrel 600 that can be used to form the first preformed curved section 400. The mandrel 600 is designed so that it imparts the desired curved shape of the distal tip section 410.

The catheter is threaded over the mandrel 600 such that the mandrel is disposed within the central lumen of the catheter. The catheter is threaded until the predetermined section of the distal end of the catheter is disposed over the curved section 601 of mandrel 600.

The catheter is then heated until the material of the catheter (e.g. PEBAX) reaches a pre-melt condition and the material becomes soft. The catheter is then allowed to cool, which allows the curve imparted by mandrel 600 to set in. After cooling, the mandrel 600 is removed. According the first curve 400 is "baked in."

The mandrel 600 can be a rod like structure that has a hook at its distal end with the hook being the structure that will define the steerable distal tip section 410. It will be appreciated that once the mandrel 600 is removed, the distal tip section 410 will spring open slightly and therefore, there is a slight difference in the shape of the mandrel 600 itself and the shape of the distal tip section 410 in the normal, rest position as shown in Fig. 13. It will be understood that Fig. 13 is only a representation of the two curves and therefore the relative dimensions of the curves in Figs. 13 and 14 vary only for illustration purposes.

Similarly, a mandrel 610 can be provided and designed to form the curved shape of the second preformed curved section 500. As mentioned above, the second preformed curved section 500 is offset and located proximal to the curved distal tip region (the first preformed curved section 500). The curve (radius of curvature) of the second curved section 500 is less pronounced than the curve of the first curved section 400 and therefore, the second curved section 500 represents a slight curve in the catheter shaft. As described below, the second curved section 500 is designed to seat against the wall of the aorta as the guide catheter 100 is used in one of its intended applications.

The mandrel 600 is thus first used to effectively "bake" in (form) the first curved section 400 at the distal end of the catheter shaft 300 and then subsequently (after the material forming the catheter shaft 300 cools), the mandrel 610 is inserted into the catheter shaft 300 for "baking" in (forming) the second curved section 500. This second process effectively forms the aortic curvature (second section 500) that is proximal to the distal tip section. Section 611 imparts the aortic curve 500 into the catheter during the bake process. Section 612 is identical in shape to section 601 of mandrel 600. Section 612 preserves the curved shape 400 during the second bake process that forms curved section 500. Section 611 and 612 can be separated by a straight section 613. Accordingly, the two curves 400, 500 of the catheter will be spaced along the catheter. The desired curvature of the distal tip section 400 is not disturbed but rather is maintained during the process for forming the aortic curved section 500.

As can be seen in Fig. 13, the second curved section 500 can impart a concave shape along a first side of the of the catheter shaft 300, while the first curved section 400 can impart a convex surface along the first side of the catheter shaft 300. Similarly, along an opposite second side of the catheter shaft 300, the first curved section 400 has a concave shape and the second curved section 500 can have a convex shape. In other words, the direction of curvature of the second curved section 500 is generally opposite the direction of curvature of the first curved section 400.

The guide catheter shaft 300 is actually formed of a number of lumen structures extend the length of the shaft 300. In particular, as shown in Figs. 12, the catheter shaft 300 has a main lumen 123 that is axially aligned with the central lumen formed in the handle body and receives an object that is to be delivered to the distal end of the guide catheter 100. The main lumen 123 is thus open at the distal end 302 of the catheter shaft 300 which in turn represents the distal end of the guide catheter 100. In addition, there is a second lumen 125 is formed adjacent the main lumen 123 and extends along a substantial length of the main lumen 123. In particular, the second lumen 125 does not extend the entire length of the main lumen 123 but instead terminates at a location that is proximal the distal end. The second lumen 125 represents a steering wire (pull wire) lumen which receives the steering wire 301 to permit the steering wire 301 to be routed to a distal end of the main lumen 123. As shown in the figure, the diameter of the second lumen 125 is less than the diameter of the main lumen 123. In the illustrated embodiment, each of the main lumen 123 and the second lumen 125 has a circular shape and is formed of suitable materials, such as PTFE.

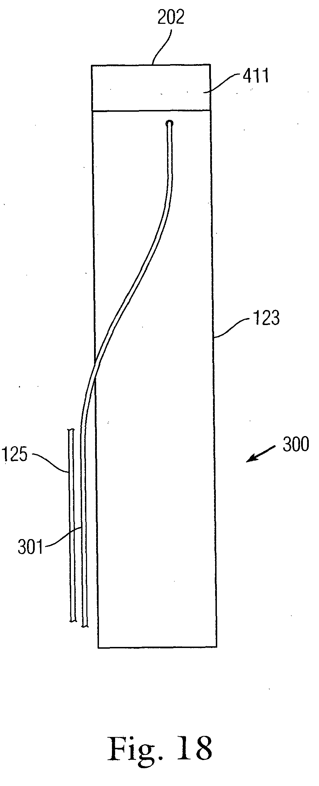

As shown in Fig. 18, the steering wire 301 exits the second lumen 125 and travels along the outer surface of the structure that defined the main lumen 123. In accordance with the present invention, the steering wire 301 does not run linearly from the end of the second lumen 125 to the distal end of the main lumen 123 but rather, the steering wire 301 is set at a degree of rotation as it runs along the length of the main lumen 123. For example and according to one exemplary embodiment, the steering wire (pull wire) 301 is rotated about 70 degrees as it advances forwardly over the main lumen 123 in the steerable distal tip section 410 (Fig. 18). The distal end of the steering wire 301 is fixed to the distal end of the main lumen 123 using conventional techniques, including bonding, etc. At the termination of the steering wire 301, a marker 309, such as a PT-IR marker band can be disposed.

In accordance with the present invention, the offsetting and rotation of the steering wire 301 permits the catheter shaft to be bent in a corkscrew manner when the steering mechanism is actuated. More specifically, the distal tip section 410 is bent in an out of plane manner unlike conventional catheter shafts which are bent in plane when the steering mechanism is actuated. The distal tip section 410 is thus bent in two planes due to the steering wire 301 being rotated about the main lumen from the point that the wire 301 exits the lumen 125 to the anchor point where the steering wire 301 is anchored to the main lumen 123. One of the planes (a first plane) is the plane that contains the distal tip section 410 and the other plane (second plane) is a plane that is out of the plane of the first plane. In other words, actuation of the steering mechanism results in the distal tip section 410 being bent not only in the first plane but also in the second plane such that the bending of the distal tip section 410 can be described as a corkscrew bending action.

Since the steering wire 301 is not fixedly attached to any structure within the second curved section 500, the second curved section 500 represents a non-steerable section. Instead, it has a generally fixed shape in a normal rest position; however, it is flexible and can be readily bent.

The corkscrew pull wire increases the maneuverability of the distal end of the catheter. Out-of-plane deflection is achieved by the pull wire is designed to correspond to the anatomy of the heart that is encountered as the catheter is being passed through the aortic valve for passage between the papillary muscles. However, it will be appreciated that the catheter 100 is not limited to deployment between the papillary muscles. The present catheter can be steered around the papillary muscles due to the above described construction. As a result, the catheter can be maneuvered to multiple points of interest on the anterior and posterior sides of the mitral valve. In addition, the catheter of the present invention is not limited to delivery to the annulus of the mitral valve. The catheter 100 of the present invention can be used to direct/deliver objects to the left ventricle wall at points located between the annulus and the papillary muscles.

It will be appreciated that a sleeve or jacket 129 covers both of the structures that define the main lumen 123 and the second lumen 125. The sleeve 129 can be formed of conventional catheter shaft materials suitable for the intended use. The steerable distal tip section 410 can have a different structure and/or be formed of a different material compared to the rest of the length of the sleeve 129. For example, the steerable distal tip section 410 can be a reinforced shaft section in the form of a coiled reinforced structure and the jacket material can be different. The jacket 129 can also extend slightly beyond the coiled reinforced structure such that a tip section 411 can be formed only of the jacket material, with the main lumen 123 being absent in this tip section 410.

A marker 417 can be provided to differentiate and mark the sleeve 129 from the reinforced distal tip section 410. The marker 417 can therefore be in the form of an annular band that is formed on the outer surface of the sleeve 129.

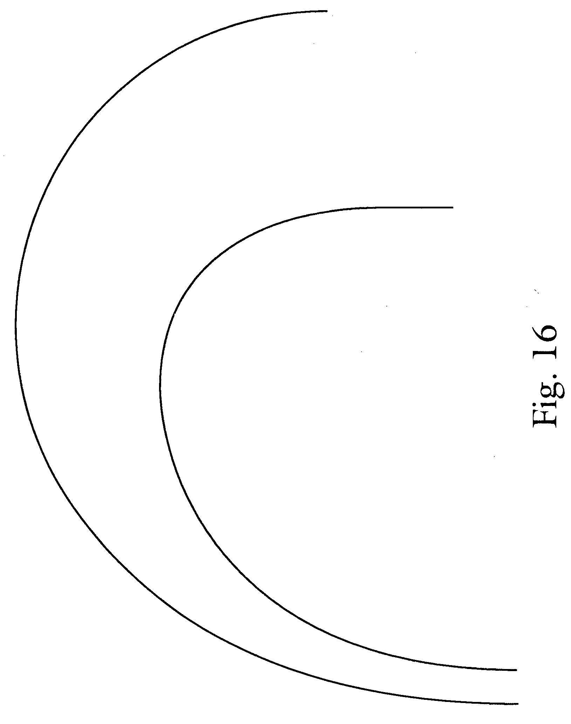

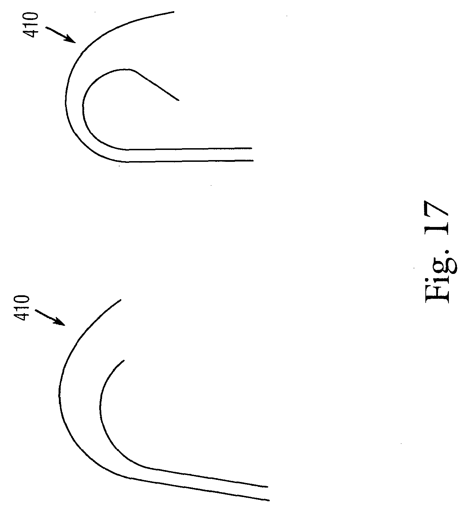

Fig. 13 generally shows the steerable distal tip section in a rest position (prior to actuation of the actuator) where the curvature and shape of the distal tip section corresponds to the preformed curved shape (the "as baked" curvature). To change the curvature of the steerable distal tip section 410 and impart a greater degree of curvature (articulation) to this section, the actuator 240 is slid within the slot 223. The sliding action of the actuator 240 (button 242) results in the steering wire 301 being pulled in a direction toward the handle body 220. This pulling action of the steering wire 301 articulates (increases the curvature) in the distal tip section 410 in that the distal tip section 410 is bent downwardly and curled towards the remaining length of catheter shaft 300 as shown in Figs. 16-17. Fig. 17 shows a range of articulation and the steerable distal tip section 410 in the maximum degree of articulation.

As mentioned above, since the steering wire 301 has a corkscrew shape in the distal tip section 410, the pulling of the steering wire 301 not only causes a strict bending of the steering wire 301 but also causes a slight rotation of the steering wire 301 as it undergoes the bending or curling motion. As can be seen in Fig. 16, second curve section 500 experiences some deflection during manipulation of the pull wire 301.

To restore the steerable distal tip section 410 to its rest position, the actuator 240 is moved (slid) in the opposite direction.

When the second curved section 500 seats against a target surface, such as an aortic wall, the rotatability of the catheter shaft 300 permits the steerable distal tip section 410 to be positioned at a desired, target location by simply rotating the handle body 220 which causes rotation of the catheter shaft 300.

This guide catheter 100 can be used as a guide sheath or tube for guiding all of the subsequent catheter devices into the left ventricle 14 for use in a method of plicating the annulus 40 of the mitral valve 20. It will be appreciated that other methods of guidance may be used as alternatives or in a supplemental fashion to the various methods disclosed herein. The steerable guide sheath is positioned at the desired location on the ventricular side of the mitral annulus for deployment of a guide wire or other instrument. For example, a guide wire can be fed through the proximal luer 360 and into the central lumen that is formed through the rotator assembly 300 and the handle body 220 and through the catheter shaft 300 itself (main lumen 123).

The guide wire can include a RF or radiofrequency energy delivery tip for assisting with penetration through the annulus tissue 40. It will be appreciated that when the "annulus tissue" is referred to herein, this refers to tissue generally along the annulus 40 and may, in fact, be tissue on the base of the posterior leaflet 24 itself. The RF guide wire is inserted through the annulus tissue 40 such that distal portions thereof extend into the left atrium 12 in manners similar to RF guide wire.

Additional details concerning guide wire catheters and guide wires and the procedure discussed above are set forth in commonly owned U.S. patent application serial No. 11/685,240, (published as U.S. patent publication No. 2008/0228265). Additional components are then used with the guide wire for attaching tissue anchors (pledgets) to tissue, such as the annulus tissue 40. For example, an anchor (pledget) delivery catheter that includes a flexible catheter body and a handle can be used for receiving and deploying a preloaded anchor as described in U.S. patent application serial No. 61/407,341 (published as U.S. patent publication No. 2012/0109155).

As previously mentioned, one particular application for the instruments disclosed herein is for use in tissue placation procedure for placating tissue; however, there are other applications as well in which the instruments can be used. For example, the guide catheter can be used to direct controlled access to a particular site (target tissue, etc.). The guide catheter disclosed herein can be used in electrophysiology (EP) applications including being used to inspect and identify bad tissue and then take remedial action using the guide catheter as a conduit through which material can be passed. More specifically, in the case in which bad tissue has been identified, the steerable guide catheter can be maneuvered and positioned at the target location and then a remedial material, such as stems cells or other molecules, can be injected through the guide catheter and delivered to the target tissue. The injection process can be coordinated and handled with an instrument (e.g., injector) that is passed through the lumen of the guide catheter. It will be appreciated that stem cells can be delivered to the damaged target tissue as a means to promote regrowth of the damaged tissue. In the case where the target tissue is stunned, the electrical pathway can be rerouted around the stunned tissue by placement of molecules that create the rerouted pathway. In addition, in situations in which the heart tissue is not cycling and firing as desired, the guide catheter can be used to provide local therapy by being able to be manipulated and positioned at the precise target site to provide such local therapy as a result of the properties of the catheter discussed herein. By using and accessing tissue that surrounds the damaged tissue, localized therapy can be provided.

As previously mentioned, the guide catheter can be used as a conduit to deliver material to a target site. The material can be in the form of stem cells, nuclear material, other molecules, or generally any other material that can provide a remedial effect. For example, the material is delivered to the tissue by means of the guide catheter being capable of being manipulated and shaped to locate and be disposed at the target tissue.

In addition, the guide catheter can be used to deliver unsheathed small gauge needles to permit internal access to tissue of interest. It will be appreciated that material, such as those mentioned above, can be delivered through the needle. In one application, leaks can occur around an artificial valve and the guide catheter of the present invention provides a means for correcting and remedying this problem by filling in the leaks around the artificial valve. In this application one or more of instruments and materials can be passed through the guide catheter once it is in place due to the advantageous structure of the guide catheter as discussed herein.

In yet another embodiment, the steerable guide catheter can be used in a transapical application. One type of transapical application is a transapical aortic valve implantation (TA-AVI), which is a relatively new therapeutic strategy that is suited for treating patients suffering symptomatic aortic stenosis and an increase perioperative risk. Transapical access is based on decades of clinical experience with de-airing the heart, through the tip (apex) of the left ventricle, during routine cardiac surgical interventions. Insertion of a catheter and later-on closure through the apex is possible in a relatively uncomplicated manner. Transapical applications can also be used to repair valves, etc. including in the manner described above with reference to the steerable guide catheter of the present invention. Thus, the steps outlined above with reference to performing tissue plication are only exemplary in nature and other techniques, including transapical techniques, can be used. In accordance with the present invention, the steerable guide catheter disclosed herein can be used in a transapical application including for valve repair, replacement and also to perform other operations, including remedying bad tissue.

It will therefore be appreciated that the guide catheter can be used to target other tissue that is of interest and provides a means for delivering not only guide wires (as discussed in detail herein) but also a means for delivering other materials and instruments as discussed above.

As a result, the devices discussed herein are not limited to tissue anchor applications and instead can be used in a number of other applications.

It will thus be appreciated that the construction of the catheter shaft is particularly suited to perform the operations described herein and more particularly, to allow for subsequently placation of the annulus of the mitral valve by delivering a guide wire that is subsequently used to deliver an instrument that is used for placating the annulus.

While the invention has been described in connection with certain embodiments thereof, the invention is capable of being practiced in other forms and using other materials and structures. Accordingly, the invention is defined by the recitations in the claims appended hereto and equivalents thereof.

## Claims

1. A steerable guide catheter (200) comprising:
a handle (210);
an elongated shaft (300), including a first main lumen (123) and a second lumen (125), extending outwardly away from and being coupled to the handle, the shaft having a distal end (202) opposite the handle, the shaft having a first curved section (400) at the distal end and a second curved section (500) that is located proximal to the first curved section; and
a steering mechanism for controllably steering at least the first curved section at the distal end, the steering mechanism being coupled between the handle and elongated shaft and being configured such that actuation of the steering mechanism causes the first curved section to bend,
**characterised in that**:
the first curved section and the second curved section are first and second preformed curved sections respectively, the second preformed curved section comprising a non-steerable section and having a concave shape, formed along a first side of the shaft, that is configured to reduce a degree of contact between the elongated shaft and the aortic arch when the elongated shaft is placed against the aortic arch, the second preformed curved section being configured to cause the catheter to bank off the aortic arch, wherein a radius of curvature of the second preformed section is less than a radius of curvature of the first preformed curved section, the first preformed curved section having a convex shape along an opposite second side of the shaft such that a direction of curvature of the second preformed curved section is generally opposite the direction of curvature of the first preformed curved section,
wherein the first preformed curved section and the steering mechanism are configured to permit the first preformed curved section to move in a corkscrew shape manner upon actuation of the steering mechanism, the second lumen (125) being parallel to the first main lumen and carries a steering wire (301) that is coupled to the steering mechanism of the handle and travels through and exits the second lumen and travels along an exterior of the first lumen such that the steering wire is set at a degree of rotation as the steering wire travels along the first lumen to a point of fixation therewith to form the corkscrew shape upon actuation of the steering mechanism.

2. The steerable guide catheter of claim 1, wherein a sleeve (129) is disposed over the first main lumen and the second lumen, the sleeve having at least two different sections with one section being located within the first preformed curved section.

3. The steerable guide catheter of claim 2, wherein the one section of the sleeve comprises a coil reinforced section.

4. The steerable guide catheter of claim 1, wherein the steering mechanism includes a slide actuator (240) including a component to which the steering wire is attached, wherein linear movement of the actuator in a proximal direction results in the steering wire being pulled and the first preformed curved section is bent to have a greater degree of curvature.

5. The steerable guide catheter of claim 4, wherein the catheter shaft is fixedly attached to a first section of the handle, the handle being formed of the first section and a second section, with the first section being rotatable relative to the second section.

6. The steerable guide catheter of claim 5, wherein the second section includes a rotator body (310) with a side port (328) extending therefrom, the rotator body being rotatably coupled to the first section, the rotator body including a lumen (330) that is in communication with and axially aligned with the main lumen to permit an object to be inserted through the rotator body and into the main lumen.

7. The steerable guide catheter of claim 6, wherein the rotator body is transparent.

8. The steerable guide catheter of claim 1, wherein the second preformed curved section is configured to seat against an aortic wall.

## Patentansprüche

1. Steuerbarer Führungskatheter (200), umfassend:
einen Griff (210);
einen länglichen Schaft (300), der ein erstes Hauptlumen (123) und ein zweites Lumen (125) umfasst, der sich nach außen, vom Griff weg erstreckt und mit diesem gekoppelt ist, wobei der Schaft ein Distalende (202) aufweist, das dem Griff entgegengesetzt ist, und wobei der Schaft einen ersten kurvenförmigen Abschnitt (400) am Distalende und einen zweiten kurvenförmigen Abschnitt (500), der in der Nähe zum ersten kurvenförmigen Abschnitt angeordnet ist, aufweist; und
einen Steuerungsmechanismus für das kontrollierbare Steuern zumindest des ersten kurvenförmigen Abschnitts am Distalende, wobei der Steuerungsmechanismus zwischen dem Griff und dem länglichen Schaft gekoppelt ist, und so konfiguriert ist, dass die Betätigung des Steuerungsmechanismus ein Biegen des ersten kurvenförmigen Abschnitts bewirkt,
**dadurch gekennzeichnet, dass**:
der erste kurvenförmige Abschnitt und der zweite kurvenförmige Abschnitt jeweils ein erster und ein zweiter vorgeformter kurvenförmiger Abschnitt sind, wobei der zweite vorgeformte kurvenförmige Abschnitt einen nicht-steuerbaren Abschnitt umfasst und eine konkave Form aufweist, die entlang einer ersten Seite des Schafts ausgebildet ist, die konfiguriert ist, ein Berührungsausmaß zwischen dem länglichen Schaft und dem Aortenbogen zu reduzieren, wenn der längliche Schaft auf dem Aortenbogen positioniert ist, wobei der zweite vorgeformte kurvenförmige Abschnitt konfiguriert ist, den Katheter zu veranlassen, sich weg vom Aortenbogen zu neigen, worin ein Krümmungsradius des zweiten vorgeformten Abschnitts geringer als ein Krümmungsradius des ersten vorgeformten kurvenförmigen Abschnitts ist, wobei der erste vorgeformte Abschnitt eine konvexe Form entlang einer entgegengesetzten zweiten Seite des Schaftes so aufweist, dass eine Krümmungsrichtung des zweiten vorgeformten kurvenförmigen Abschnitts der Krümmungsrichtung des ersten vorgeformten kurvenförmigen Abschnitts allgemein entgegengesetzt ist,
worin der erste vorgeformte kurvenförmige Abschnitt und der Steuerungsmechanismus konfiguriert sind, es dem ersten vorgeformten kurvenförmigen Abschnitt zu ermöglichen, sich in einer korkenzieherförmigen Art und Weise bei Betätigung des Steuerungsmechansimus zu bewegen, wobei das zweite Lumen (125) parallel zum ersten Hauptlumen ist und einen Steuerungsdraht (301) mit sich führt, der mit dem Steuerungsmechanismus des Griffs gekoppelt ist und sich hindurchbewegt und aus dem zweiten Lumen austritt und sich an einer Außenseite des ersten Lumens so entlangbewegt, dass der Steuerungsdraht bei einem Rotationsausmaß eingestellt wird, wenn sich der Steuerungsdraht am ersten Lumen zu einem Befestigungspunkt damit entlangbewegt, um die Korkenzieherform bei Betätigung des Steuerungsmechanismus auszubilden.

2. Steuerbarer Führungskatheter nach Anspruch 1, worin eine Hülse (129) über das erste Hauptlumen und das zweite Lumen angeordnet ist, wobei die Hülse mindesten zwei unterschiedliche Abschnitte aufweist, von denen ein Abschnitt innerhalb des ersten vorgeformten kurvenförmigen Abschnitts positioniert ist.

3. Steuerbarer Führungskatheter nach Anspruch 2, worin der eine Abschnitt der Hülse einen Spulen-verstärkten Abschnitt umfasst.

4. Steuerbarer Führungskatheter nach Anspruch 1, worin der Steuerungsmechanismus einen Gleit-Aktuator (240) umfasst, der eine Komponente umfasst, an welcher der Steuerungsdraht festgemacht ist, worin eine lineare Bewegung des Aktuators in eine proximale Richtung dazu führt, dass der Steuerungsdraht gezogen wird, und der erste vorgeformte kurvenförmige Abschnitt verbogen wird, um ein größeres Krümmungsausmaß aufzuweisen.

5. Steuerbarer Führungskatheter von Anspruch 4, worin der Katheter-Schaft fix an einem ersten Abschnitt des Griffs festgemacht ist, wobei der Griff aus dem ersten Abschnitt und einem zweiten Abschnitt ausgebildet ist, und wobei der erste Abschnitt relativ zum zweiten Abschnitt drehbar ist.

6. Steuerbarer Führungskatheter nach Anspruch 5, worin der zweite Abschnitt einen Rotatorkörper (310) mit einer Seitenöffnung (328), die sich von diesem erstreckt, umfasst, wobei der Rotatorkörper mit dem ersten Abschnitt drehbar gekoppelt ist, und wobei der Rotatorkörper ein Lumen (330) umfasst, das in Kommunikation mit dem Hauptlumen, und mit diesem axial ausgerichtet ist, um zu ermöglichen, dass ein Objekt durch den Rotatorkörper hindurch, und in das Hauptlumen hinein, eingebracht wird.

7. Steuerbarer Führungskatheter von Anspruch 6, worin der Rotatorkörper transparent ist.

8. Steuerbarer Führungskatheter nach Anspruch 1, worin der zweite vorgeformte kurvenförmige Abschnitt konfiguriert ist, an einer Aortenwand anzuliegen.

## Revendications

1. Cathéter-guide orientable (200), comprenant :
une poignée (210) ;
un arbre allongé (300), comprenant une première lumière principale (123) et une seconde lumière (125), s'étendant vers l'extérieur à l'opposé de, et étant couplé à la poignée, l'arbre ayant une extrémité distale (202) opposée à la poignée, l'arbre ayant une première section incurvée (400) à l'extrémité distale et une seconde section incurvée (500) qui est située à proximité de la première section incurvée ; et
un mécanisme de direction pour diriger de façon commandée au moins la première section incurvée à l'extrémité distale, le mécanisme de direction étant couplé entre la poignée et l'arbre allongé et étant configuré de telle sorte que l'actionnement du mécanisme de direction amène la première section incurvée à s'incurver,
**caractérisé en ce que** :
la première section incurvée et la seconde section incurvée sont des première et seconde sections incurvées préformées, respectivement, la seconde section incurvée préformée comprenant une section non orientable et ayant une forme concave, formée le long d'un premier côté de l'arbre, qui est configurée pour réduire une degré de contact entre l'arbre allongé et l'arc aortique lorsque l'arbre allongé est placé contre l'arc aortique, la seconde section incurvée préformée étant configurée pour amener le cathéter à renforcer l'arc aortique, dans lequel un rayon de courbure de la seconde section préformée est inférieur à un rayon de courbure de la première section incurvée préformée, la première section incurvée préformée ayant une forme convexe le long d'un deuxième côté opposé de l'arbre de telle sorte qu'une direction de courbure de la deuxième section incurvée préformée est généralement opposée à la direction de courbure de la première section incurvée préformée,
dans lequel la première section incurvée préformée et le mécanisme de direction sont configurés pour permettre à la première section incurvée préformée de se déplacer d'une manière en forme de tire-bouchon lors de l'actionnement du mécanisme de direction, la deuxième lumière (125) étant parallèle à la première lumière principale, et porte une fil de guidage (301) qui est couplé au mécanisme de direction de la poignée, et se déplace à travers et sort de la deuxième lumière et se déplace le long d'un extérieur de la première lumière de telle sorte que le fil de guidage est fixé à un degré de rotation tel que le fil de guidage se déplace le long de la première lumière jusqu'à un point de fixation avec celle-ci pour former la forme de tire-bouchon lors de l'actionnement du mécanisme de direction.

2. Cathéter de guidage orientable selon la revendication 1, dans lequel un manchon (129) est disposé au-dessus de la première lumière principale et de la seconde lumière, le manchon ayant au moins deux sections différentes, une section étant située à l'intérieur de la première section incurvée préformée.

3. Cathéter de guidage orientable selon la revendication 2, dans lequel la première section du manchon comprend une section de bobine renforcée.

4. Cathéter de guidage orientable selon la revendication 1, dans lequel le mécanisme de direction comprend un actionneur coulissant (240) comprenant un composant auquel le fil de guidage est relié, dans lequel un mouvement linéaire de l'actionneur dans une direction proximale a pour résultat que le fil de guidage est tiré, et la première section incurvée préformée est incurvée pour avoir un plus grand degré de courbure.

5. Cathéter de guidage orientable selon la revendication 4, dans lequel l'arbre de cathéter est attaché de façon fixe à une première section de la poignée, la poignée étant formée de la première section et d'une seconde section, la première section étant mobile en rotation par rapport à la seconde section.

6. Cathéter de guidage orientable selon la revendication 5, dans lequel la seconde section comprend un corps de rotateur (310) avec un orifice latéral (328) s'étendant à partir de celle-ci, le corps de rotateur étant couplé de manière rotative à la première section, le corps de rotation comprenant une lumière (330) qui est en communication et axialement alignée avec la lumière principale pour permettre à un objet d'être inséré à travers le corps de rotation et jusque dans la lumière principale.

7. Cathéter de guidage orientable selon la revendication 6, dans lequel le corps de rotateur est transparent.

8. Cathéter de guidage orientable selon la revendication 1, dans lequel la seconde section incurvée préformée est configurée pour prendre appui contre une paroi aortique.
